Europäisches Patentamt

European Patent Office

Office européen des brevets

(11) Veröffentlichungsnummer: **0 269 844 B1**

## (12) EUROPÄISCHE PATENTSCHRIFT

(45) Veröffentlichungstag der Patentschrift: **16.06.93**

(21) Anmeldenummer: **87115678.2**

(22) Anmeldetag: **26.10.87**

(51) Int. Cl.⁵: **A61N 1/08**

---

54 **Reizstromgerät.**

---

(30) Priorität: **07.11.86 DE 3638001**

(43) Veröffentlichungstag der Anmeldung:
**08.06.88 Patentblatt 88/23**

(45) Bekanntmachung des Hinweises auf die
Patenterteilung:
**16.06.93 Patentblatt 93/24**

84 Benannte Vertragsstaaten:
**AT BE CH DE FR LI NL**

56 Entgegenhaltungen:
**EP-A- 0 026 479**
**FR-A- 2 507 900**
**GB-A- 2 085 593**

73 Patentinhaber: **SIEMENS AKTIENGESELL-**
**SCHAFT**
**Wittelsbacherplatz 2**
**W-8000 München 2(DE)**

72 Erfinder: **Helmreich, Klaus**
**Rhönstrasse 36**
**W-8520 Erlangen(DE)**
Erfinder: **Herzog, Ludwig**
**Drei-Thorn-Strasse 3**
**W-6948 Waldmichelbach(DE)**
Erfinder: **Knapp, Volker**
**Pestalozzistrasse 19**
**W-6948 Waldmichelbach(DE)**

**Beschreibung**

Die Erfindung bezieht sich auf ein Reizstromgerät gemäß Oberbegriff des Patentanspruchs 1.

Ein Gerät dieser Art mit einer Sicherheitsschaltung für Konstantstrombetrieb (CC) ist z.B. aus der EP-B-0 026 324 bekannt.

Aus der FR-A-2 507 900 ist ein Reizstromgerät mit einer Gefahrmeldeschaltung bekannt, die ein Alarmsignal erzeugt, wenn ein unzulässig hoher Strom fließt. Das Alarmsignal wird aus mehreren Vergleichsschaltungen erzeugt, wobei ein kontinuierlich gemessener Istwert in mehreren Vergleichsschaltungen mit mehreren Vergleichswerten verglichen wird. Die Vergleichswerte werden aus dem Istwert durch Spannungsteilung über Widerstände und durch Invertierung erzeugt.

Aufgabe vorliegender Erfindung ist es, ein Reizstromgerät mit Sicherheitsschaltung aufzubauen, die technisch einfach ist und auch zur Stromabschaltung bei Konstantspannungsbetrieb (CV), wo bekanntlich auf Grund sich ändernden Patientenwiderstandes der Strom stark ansteigen kann, dient.

Die Aufgabe wird erfindungsgemäß bei einem Reizstromgerät der eingangs genannten Art durch die kennzeichnenden Merkmale des Patentanspruchs 1 gelöst.

Bei einem Reizstromgerät gemäß der Erfindung wird abgeschaltet, sobald der Istwert ein vorgebbares (geringes) Vielfaches (z.B. 1.2) des Referenzstromwertes beträgt. Die Schaltung ist einfach und läßt sich sowohl bei Konstantstrom als auch bei Konstantspannungsbetrieb einsetzen.

Weitere Vorteile und Einzelheiten der Erfindung ergeben sich aus der nachfolgenden Beschreibung eines Ausführungsbeispieles anhand der Zeichnung und in Verbindung mit den Unteransprüchen.

Es zeigen:

Fig. 1     das erfindungsgemäße Reizstromgerät im Prinzipschaltbild und

Fig. 2     ein Diagramm der Transformationsmatrix der Stromtransformationseinrichtung.

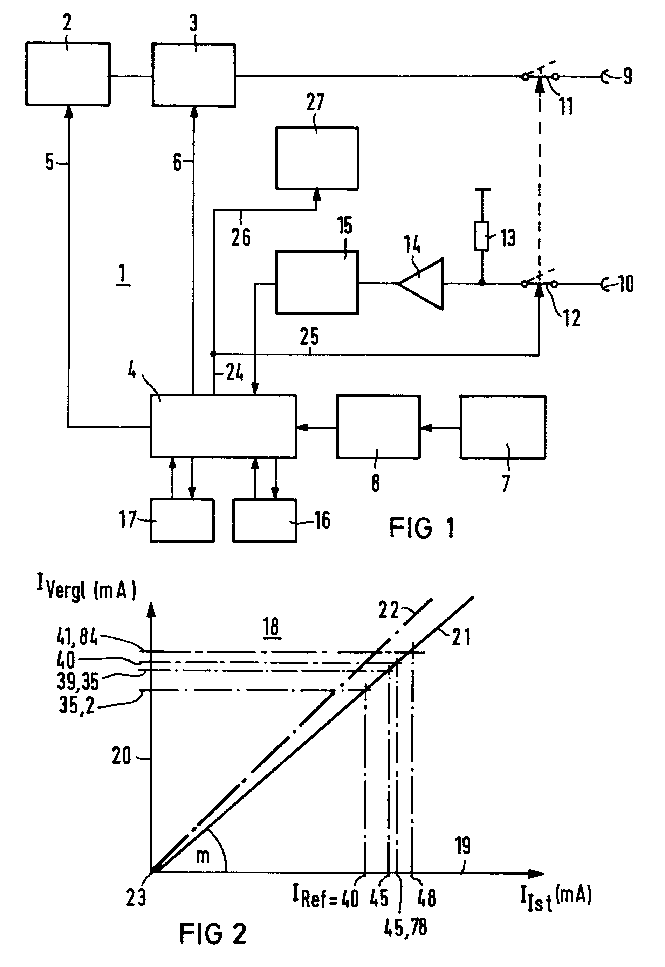

In der Fig. 1 umfaßt das Reizstromgerät 1 unter anderem einen Reizstromerzeuger 2 mit Intensitätseinstelleinrichtung 3, die von einem Mikroprozessor 4 über Leitungen 5, 6 gesteuert werden. Der Intensitätseinstelleinrichtung 3 ist dabei über den Mikroprozessor 4 ein Intensitätseinstellglied 7 mit vorgeschaltetem Analog-Digital-Wandler 8 zugeordnet. Die Buchsen 9, 10 sind die Geräteanschlußbuchsen für die (nicht dargestellten) Reizstromelektroden. Die (Relais) Schaltkontakte 11, 12 unterbrechen bei Betätigung (gestrichelte Stellung) den inneren Stromkreis des Reizstromgerätes 1. Damit kann bei Unterbrechung kein Reizstrom zum über die Elektroden angeschalteten Patienten gelangen.

Zur Messung der Istwerte des Stromes beinhaltet das Reizstromgerät 1 ferner einen Meßwiderstand 13, dem über einen Verstärker 14 ein Analog-Digital-Wandler 15 nachgeschaltet ist. Der Analog-Digital-Wandler 15 tastet, gesteuert durch den Mikroprozessor 4, den am Widerstand 13 anfallenden stromproportionalen Spannungsabfall ca. 20 mal pro Sekunde ab (Istwerte des Stromes).

Bei Konstantspannungsbetrieb wird nach jeder Betätigung des Intensitätseinstellgliedes 7 vom Mikroprozessor 4 zuerst der sich neu einstellende Stromspitzenwert aus den anfallenden Istwerten ermittelt und in einem RAM 16 als Erstwert abgespeichert. Dieser gespeicherte Erstwert dient dann in der Folge als Referenzstromwert.

Bei Konstantstrombetrieb kann der Stromsollwert direkt als Referenzstromwert in das RAM 16 eingespeichert sein.

Wesentlich für die vorliegende Erfindung ist nun der Aufbau der Stromtransformationsmatrix, die in einem mit dem Mikroprozessor 4 in Dialog stehenden EPROM 17, das hier als Stromtransformationseinrichtung arbeitet, eingespeichert ist.

Die Fig. 2 zeigt eine solche Stromtransformationsmatrix 18 im Detail.

Gemäß der Fig. 2 sind entlang der Abszisse 19 die Istwerte $I_{Ist}$ (aktuelle Meßwerte) in mA aufgetragen. Längs der Ordinate 20 finden sich die transformierten Vergleichsstromwerte $I_{Vergl}$ ebenfalls in mA. Die Transformation $I_{Ist}$ nach $I_{Vergl}$ ergibt sich mittels einer Transformationsgeraden 21, deren Steigung unterhalb der 45°-Geraden 22 einen Wert m aufweist, der etwa dem reziproken Wert des Vielfachen des Referenzstromwertes $I_{Ref}$ entspricht, bei dessen Erreichen der innere Stromkreis abgeschaltet werden soll.

Im vorliegenden Fall soll z.B. abgeschaltet werden, wenn der Istwert des Stromes ungefähr das 1,2-fache des Referenzstromwertes $I_{Ref}$ erreicht. Die Steigung der Transformationsgeraden beträgt also im vorliegenden Ausführungsbeispiel z.B. m = 0,83 (1:m = 1,2). Zur Vermeidung von Meßunsicherheiten beginnt die Transformationsgerade 21 im vorliegenden Fall nicht im Koordinatenursprung 23, sondern z.B. um zwei Digits nach rechts verschoben auf der Abszisse. Die Transformationsgerade 21 verläuft dann also z.B. nach der Beziehung $I_{Vergl}$ = 0,83 $I_{Ist}$ + 2.

Im vorliegenden Ausführungsbeispiel sei der momentane Referenzstromwert beispielsweise $I_{Ref}$ = 40 mA (für CC Konstantstromwert, bei CV jedoch variabel nach jeder Intensitätseinstellung neu zu messender Wert). Gemäß der Fig. 2 ergibt sich ein Vergleichsstromwert $I_{Vergl}$ = 40 mA, wenn $I_{Ist}$ = 45,78 mA (oder $I_{Ist}$ = 1,14 x $I_{Ref}$). Jeder Istwert $I_{Ist}$ größer 45,78 mA führt also zur Abschaltung. Bei $I_{Ist}$ = 48 mA, was exakt dem 1,2-fachen des Referenzstromwertes $I_{Ref}$ = 40 mA entspricht (1,2-facher Stromanstieg), ist der innere Stromkreis also immer mit Sicherheit vom Patienten abgeschaltet.

Der Vergleich eines Vergleichsstromwertes $I_{Vergl}$ (transformierter Istwert) mit dem Referenzstromwert $I_{Ref}$ (gespeichert im RAM 16) erfolgt durch den Mikroprozessor 4. Sobald der Mikroprozessor 4 feststellt, daß $I_{Vergl}$ größer $I_{Ref}$, erzeugt er an einem Mikroprozessor-Ausgang 24 ein Signal, das über die Leitung 25 als Abschaltsignal die (Relais) Kontakte 11, 12 im Sinne einer Öffnung aktiviert, wodurch - wie zuvor schon erwähnt - der innere Stromkreis des Reizstromgerätes 1 abgeschaltet wird. Gleichzeitig stößt das Signal auch über eine Leitung 26 einen optischen und akustischen Alarmsignalgeber 27 im Sinne einer Alarmauslösung an.

**Patentansprüche**

1. Reizstromgerät, insbesondere für Reizstromtherapie an einem Patienten, mit einem Reizstromerzeuger, dessen Intensität einstellbar ist, und mit einer Sicherheitsschaltung, die den inneren Stromkreis abschaltet, sobald der Reizstrom in vorgebbarem Maße von einem vorgegebenen Referenzstromwert abweicht, **gekennzeichnet durch** eine Stromabtasteinrichtung (13 bis 15), welche digitale Istwerte ($I_{Ist}$) des Stromes an eine einen Festwertspeicher (17) umfassende Stromtransformationseinrichtung (4, 17) abgibt, die abgetastete Istwerte in Vergleichsstromwerte ($I_{Vergl}$) transformiert, die um einen vorgebbaren Bruchteil niedriger sind als die Istwerte und eine Vergleichseinrichtung (4), die Vergleichsstromwerte mit dem Referenzstromwert ($I_{Ref}$) vergleicht, wobei in dem Festwertspeicher (17) eine Stromtransformationsmatrix (18) gespeichert ist, die auf der Abszisse (19) die Istwerte ($I_{Ist}$) des Stromes und auf der Ordinate (20) die Vergleichsstromwerte ($I_{Vergl}$) umfaßt und die die Istwerte zu den Vergleichsstromwerten mittels einer Transformationsgeraden (21) transformiert, deren Steigung unterhalb der 45°-Geraden (22) einen Wert (m) aufweist, der etwa dem reziproken Wert des Vielfachen des Referenzstromwertes entspricht, bei dessen Erreichen oder Überschreiten die Vergleichseinrichtung (4) ein Abschaltsignal (11, 12) für den inneren Stromkreis erzeugt.

2. Reizstromgerät nach Anspruch 1, **dadurch gekennzeichnet**, daß im Konstantstrombetrieb der Referenzstromwert durch den Stromsollwert vorgegeben ist.

3. Reizstromgerät nach Anspruch 1, **dadurch gekennzeichnet**, daß für Konstantspannungsbetrieb der Stromabtasteinrichtung (13 bis 15) ein Erstwertspeicher (16) zugeordnet ist, der den Spitzenwert eines nach Einstellung der Intensität erhaltenen Reizstromes als Erstwert speichert und daß der Erstwert der Referenzstromwert ist.

4. Reizstromgerät nach Anspruch 3, **dadurch gekennzeichnet**, daß der Erstwertspeicher (16) ein RAM ist.

5. Reizstromgerät nach Anspruch 2, **dadurch gekennzeichnet**, daß der Stromsollwert in einem RAM (16) gespeichert ist.

6. Reizstromgerät nach einem der Ansprüche 1 bis 5, **dadurch gekennzeichnet**, daß der Festwertspeicher (17) ein EPROM ist.

7. Reizstromgerät nach Anspruch 1, **dadurch gekennzeichnet**, daß die Transformationsgerade (21) nicht im Koordinatenursprung (23) der Stromtransformationsmatrix, sondern um einige Digits (n) in Richtung der Abszisse verschoben beginnt ($I_{Vergl}$ = m $I_{Ist}$ + n).

8. Reizstromgerät nach Anspruch 7, **dadurch gekennzeichnet**, daß die Gerade nach der Beziehung $I_{Vergl}$ = 0,83 $I_{Ist}$ + 2 verläuft.

9. Reizstromgerät nach einem der Ansprüche 1 bis 8, **dadurch gekennzeichnet**, daß die Stromabtasteinrichtung einen Strommeßwiderstand (13) und einen nachgeschalteten Analog-Digital-Wandler (15) für die Istwerte des Stromes umfaßt, und daß die Vergleichseinrichtung (4) ein Mikroprozessor ist, der anfallende Istwerte nach Transformation in der Transformationseinrichtung (17) zu Vergleichsstromwerten mit dem Referenzstromwert vergleicht und das Abschaltsignal für den inneren Stromkreis erzeugt, sobald ein Vergleichsstromwert den Referenzstromwert überschreitet.

**Claims**

1. Current stimulator, in particular for current stimulation therapy on a patient, having a stimulating-current producer, the intensity of which is adjustable, and having a safety circuit which disconnects the inner current circuit as soon as the stimulating current deviates to a specifiable extent from a specified current reference value, characterized by a current-sampling device (13 to 15) which delivers digital actual values ($I_{Actual}$) of the current to a current-transformation device (4, 17) comprising a fixed-value memory (17), which device transforms sampled actual values into current comparison values ($I_{Comp}$), which are lower by a specifiable fraction than the actual values and a comparison device (4) which compares current comparison values with the current reference value ($I_{Ref}$), whereby a current-transformation matrix (18) is stored in the fixed-value memory (17), which matrix on the abscissa (19) comprises the actual values ($I_{Actual}$) of the current and on the ordinate (20) the current comparison values ($I_{Comp}$) and which transforms the actual values into the current comparison values by means of a transformation line (21), the gradient of which below the 45° line (22) has a value (m) which corresponds approximately with the reciprocal value of the multiple of the current reference value, upon whose reaching or exceeding the comparison device (4) generates a disconnection signal (11, 12) for the inner current circuit.

2. Current stimulator according to claim 1, characterized in that in the constant current operation the current reference value is specified by the desired current value.

3. Current stimulator according to claim 1, characterized in that for constant voltage operation there is associated with the current-sampling device (13 to 15) a first-value memory (16), which stores as a first value the peak value of a stimulating current obtained after the adjustment of the intensity and in that the first value is the current reference value.

4. Current stimulator according to claim 3, characterized in that the first-value memory (16) is a RAM.

5. Current stimulator according to claim 2, characterized in that the desired current value is stored in a RAM (16).

6. Current stimulator according to one of claims 1 to 5, characterized in that the fixed-value memory (17) is an EPROM.

7. Current stimulator according to claim 1, characterized in that the transformation line (21) does not begin in the coordinate origin (23) of the current-transformation matrix but displaced by a few digits (n) in the direction of the abscissa ($I_{Comp} = m\ I_{Actual} + n$).

8. Current stimulator according to claim 7, characterized in that the line runs according to the equation $I_{Comp} = 0.83\ I_{Actual} + 2$.

9. Current stimulator according to one of claims 1 to 8, characterized in that the current-sampling device comprises a current-measuring resistance (13) and a subsequently connected analog-to-digital converter (15) for the actual values of the current, and in that the comparison device (4) is a microprocessor which compares actual values obtained after transformation in the transformation device (17) into current comparison values with the current reference value and generates the disconnection signal for the inner current circuit as soon as a current comparison value exceeds the current reference value.

## Revendications

1. Simulateur, notamment pour la thérapie à courant de stimulation appliquée à un patient, comportant un générateur de courant de stimulation, dont l'intensité est réglable, et un circuit de sécurité qui débranche le circuit interne dès que le courant d'excitation s'écarte, d'une valeur pouvant être prédéterminée, d'une valeur de référence prédéterminée, caractérisé par un dispositif d'échantillonnage du courant (13 à 15), qui envoie des valeurs réelles numériques ($I_{Ist}$) du courant à un dispositif de transformation du courant (4,17), qui comprend une mémoire morte (17) et qui transforme les valeurs réelles échantillonnées en valeurs de courant de comparaison ($I_{vergl}$) qui sont plus faibles, d'une fraction pouvant être prédéterminée, aux valeurs réelles, et un dispositif comparateur (4), qui compare les valeurs du courant de comparaison à la valeur du courant de référence ($I_{Ref}$),auquel cas dans la mémoire morte (17) est mémorisée une matrice de transformation de courant (18), qui comporte, en abscisses (19), les valeurs réelles ($I_{Ist}$) du courant et, en ordonnées (20), les valeurs du courant de comparaison ($I_{vergl}$), et transforme les valeurs réelles en les valeurs de courant de comparaison au moyen d'une droite de transformation (21), dont la pente possède, au-dessous de la droite à 45° (22), une valeur (m) qui correspond approximativement à l'inverse du quadruple de la valeur du courant de référence, le dispositif comparateur (4) produisant un signal de débranchement (11,12) pour le circuit intérieur lorsque ladite valeur est atteinte ou dépassée.

2. Simulateur suivant la revendication 1, caractérisé par le fait que dans le cas du fonctionnement à courant constant, la valeur du courant de référence est prédéterminée par la valeur de consigne du courant.

4

3. Stimulateur suivant la revendication 1, caractéirsé par le fait que pour le fonctionnement à tension constante, au dispositif d'échantillonnage de courant (13 à 15) est associée une mémoire de première valeur (16), qui mémorise, en tant que première valeur, la valeur maximale d'un courant d'excitation produit après réglage de l'intensité, et que la première valeur est la valeur du courant de référence.

4. Stimulateur suivant la revendication 3, caractérisé par le fait que la mémoire de première valeur (16) est une mémoire RAM.

5. Stimulateur suivant la revendication 2, caractérisé par le fait que la valeur de consigne du courant est mémorisée dans une mémoire RAM (16).

6. Stimulateur suivant l'une des revendications 1 à 5, caractérisé par le fait que la mémoire morte (17) est une mémoire EPROM.

7. Stimulateur suivant la revendication 1, caractérisé par le fait que la droite de transformation (21) commence non pas à l'origine (23) des coordonnées de la matrice de transformation de courant, mais en étant décalée de quelques chiffres (n) dans la direction des abscisses ($I_{Vergl} = m I_{Ist} + n$).

8. Stimulateur suivant la revendication 7, caractérisé par le fait que la droite correspond à la relation $I_{Vergl} = 0,83 \, I_{Ist} + 2$.

9. Stimulateur suivant l'une des revendications 1 à 8, caractérisé par le fait que le dispositif d'échantillonnage du courant comprend une résistance ampèremétrique (9) et un convertisseur analogique/numérique (15) branché en aval, pour les valeurs réelles du courant, et que le dispositif comparateur (4) est un microprocesseur, qui compare des valeurs réelles arrivantes, après leur transformation dans le dispositif de transformation (17) en des valeurs de courant de comparaison, à la valeur du courant de référence et produit le signal de débranchement pour le circuit interne dès qu'une valeur de courant de comparaison dépasse la valeur du courant de référence.

FIG 1

FIG 2